# EUROPÄISCHE PATENTSCHRIFT

(11) **EP 4 279 028 B1**
(45) Veröffentlichungstag und Bekanntmachung des Hinweises auf die Patenterteilung: **21.08.2024**
(21) Anmeldenummer: 23171867.7
(22) Anmeldetag: 05.05.2023
(51) Int. Cl.: A61F 2/16, A61L 27/00

(54) **INTRAOKULARLINSE**
INTRAOCULAR LENS
LENTILLE INTRAOCULAIRE

(30) Priorität: 20.05.2022 DE 102022112805
(43) Veröffentlichungstag der Anmeldung: 22.11.2023
(73) Patentinhaber: Carl Zeiss Meditec AG, 07745 Jena (DE)
(72) Erfinder: KIRCHNER, Friedrich, 73447 Oberkochen (DE)
(74) Vertreter: PATERIS Patentanwälte PartmbB

(56) Entgegenhaltungen:
- WO-A1-2021/018533
- WO-A1-90/11736

## Beschreibung

Die Erfindung betrifft eine Intraokularlinse.

Bei einer Kataraktbehandlung wird die natürliche Linse eines menschlichen Auges durch eine künstliche Intraokularlinse ersetzt. Dazu wird die natürliche Linse beispielsweise mittels Phakoemulsifikation zerkleinert und anschließend aus einem Kapselsack des Auges abgesaugt. Bei einer extrakapsulären Katarakt-Extraktion verbleibt der Kapselsack der Linse dabei im Auge. Die Intraokularlinse weist einen Optikkörper auf, welcher in der Regel eine im Wesentlichen diskusartige Form besitzt. Dadurch kann der Kapselsack in seinem hinteren Bereich die Intraokularlinse beziehungsweise deren Optikkörper berühren. Dies kann dazu führen, dass mehrere Monate oder sogar Jahre nach der Kataraktbehandlung ein sogenannter Nachstar entstehen kann, der zu einer Trübung des Kapselsacks in dem hinteren Bereich führt. Die Trübung geht einher mit einer Verschlechterung des Sehens. Der Nachstar kann durch eine sogenannte Kapsulotomie behandelt werden. Dabei wird der eingetrübte hintere Kapselsack beispielsweise mit mehreren Impulsen von Laserstrahlung eröffnet, so dass eine Verbesserung des Sehens erreicht wird. Wünschenswert wäre es jedoch, wenn der Nachstar nach der Kataraktbehandlung gar nicht erst entsteht. DE 10 2019 211 435 B3 offenbart eine Intraokularlinse mit einer spreizbaren Haptik gemäß dem Oberbegriff des Anspruchs 1. US 3 994 027 A offenbart eine präpupilläre Linse zum Implantieren in ein menschliches Auge. WO 90/ 11 736 A1 offenbart eine Intraokularlinse mit einer expandierbaren Haptik.

Aufgabe der Erfindung ist es daher, eine Intraokularlinse zu schaffen, bei der die Wahrscheinlichkeit eines Entstehens eines Nachstars nach einer Kataraktbehandlung gering ist.

Die erfindungsgemäße Intraokularlinse weist einen Optikkörper, eine erste Haptik und einen Superabsorber auf. Der Optikkörper weist eine optische Achse auf. Die erste Haptik weist eine erste Teilhaptik und eine zweite Teilhaptik auf. Die erste Teilhaptik und die zweite Teilhaptik sind an dem Optikkörper befestigt und in einer Axialrichtung bezüglich der optischen Achse nebeneinander angeordnet. Zudem haben die erste Teilhaptik und die zweite Teilhaptik einen Annäherungszustand und einen Beabstandungszustand, in dem die erste Teilhaptik und die zweite Teilhaptik weiter voneinander entfernt als in dem Annäherungszustand sind. Der Superabsorber ist in der Axialrichtung zwischen der ersten Teilhaptik und der zweiten Teilhaptik angeordnet und eingerichtet, sein Volumen durch eine Absorption von Wasser zu vergrößern und somit die erste Teilhaptik und die zweite Teilhaptik von dem Annäherungszustand in den Beabstandungszustand zu bringen.

Wenn die Intraokularlinse während einer Kataraktbehandlung in einen Kapselsack eines Auges eingebracht wird, kann der Superabsorber in dem Kapselsack befindliches Wasser absorbieren. Dadurch vergrößert sich das Volumen des Superabsorbers, wodurch die erste Haptik von dem Annäherungszustand in den Beabstandungszustand gelangt. In dem Beabstandungszustand können die erste Teilhaptik und die zweite Teilhaptik den Kapselsack aufspannen, wodurch eine Linsenkapsel mindestens in einem hinteren Bereich des Kapselsacks beabstandet von dem Optikkörper gehalten werden kann. Dadurch kann das in dem Kapselsack befindliche Wasser trotz eines diskusartigen Optikkörpers gut zirkulieren. Dadurch, dass die Linsenkapsel beabstandet von dem Optikkörper ist, und dadurch, dass das Wasser besser zirkulieren kann, ist eine Wahrscheinlichkeit gering, dass sich nach der Kataraktbehandlung ein Nachstar bildet.

In dem Beabstandungszustand ragen die erste Teilhaptik und die zweite Teilhaptik bevorzugt zumindest teilweise in der Axialrichtung über den Optikkörper hinaus. Dadurch kann sichergestellt werden, dass die Linsenkapsel den Optikkörper nicht kontaktiert, wodurch die Wahrscheinlichkeit für eine Bildung eines Nachstars besonders gering ist.

Es ist bevorzugt, dass die Intraokularlinse eine gedachte Trennebene aufweist, deren Normale parallel zu der Axialrichtung ist, wobei die erste Teilhaptik im Wesentlichen vollständig auf einer Seite der Trennebene angeordnet ist und die zweite Teilhaptik im Wesentlichen vollständig auf einer anderen Seite der Trennebene angeordnet ist. Dies gilt besonders bevorzugt, sowohl wenn sich die erste Teilhaptik und die zweite Teilhaptik in dem Annäherungszustand befinden als auch wenn sich die erste Teilhaptik und die zweite Teilhaptik in dem Beabstandungszustand befinden. Insbesondere gilt dies auch, wenn sich die Intraokularlinse in einem Entspannungszustand befindet, in dem die Intraokularlinse frei von einer mechanischen Spannung ist und sich die erste Teilhaptik und die zweite Teilhaptik in dem Annäherungszustand befinden. Aufgrund einer Rauheit der Oberfläche der ersten Teilhaptik und/oder der zweiten Teilhaptik können sich erste Teilhaptik und/oder die zweite Teilhaptik geringfügig auch auf die Seite der jeweils anderen Teilhaptik erstrecken.

Es ist bevorzugt, dass die erste Teilhaptik ein erstes Längsende aufweist, das dem Optikkörper abgewandt angeordnet ist, und die zweite Teilhaptik ein zweites Längsende aufweist, das dem Optikkörper abgewandt angeordnet ist, wobei in dem Annäherungszustand die erste Teilhaptik in einem Bereich des ersten Längsendes die zweite Teilhaptik in einem Bereich des zweiten Längsendes kontaktiert, wobei in dem Beabstandungszustand die erste Teilhaptik in dem Bereich des ersten Längsendes beabstandet von der zweiten Teilhaptik in dem Bereich des zweiten Längsendes angeordnet ist. Indem die erste Teilhaptik die zweite Teilhaptik kontaktiert, nimmt die Intraokularlinse in dem Annäherungszustand nur einen geringen Raum ein. Dadurch kann die Intraokularlinse in eine besonders kleine Packung gefaltet werden, wenn die Intraokularlinse mittels eines Injektors in den Kapselsack eingeführt wird. Dadurch kann ein Schnitt in einer Hornhaut des Auges, via den die Intraokularlinse eingeführt wird, besonders klein ausgeführt werden.

Die erste Teilhaptik weist bevorzugt an seiner der zweiten Teilhaptik zugewandten Seite eine erste Aussparung auf, in der der Superabsorber angeordnet ist. Die erste Aussparung hat besonders bevorzugt einen halbkreisförmigen Querschnitt. Zudem weist die zweite Teilhaptik bevorzugt eine zweite Aussparung auf, in der der Superabsorber angeordnet ist. Die zweite Aussparung hat besonders bevorzugt einen halbkreisförmigen Querschnitt.

Die Intraokularlinse weist bevorzugt einen Ring auf, der sich in einer Umfangsrichtung bezüglich der optischen Achse vollumfänglich um den Optikkörper erstreckt und der den Superabsorber aufweist. Dadurch, dass der Ring zwischen der ersten Teilhaptik und der zweiten Teilhaptik angeordnet ist und sich vollumfänglich um den Optikkörper erstreckt, ist der Ring vorteilhaft besonders fest an der verbliebenen Intraokularlinse angebracht.

Die Intraokularlinse weist bevorzugt eine Hülle auf, die für das Wasser durchlässig ist und in der der Superabsorber angeordnet ist, wobei die Hülle einen Lagerungszustand, in dem der Superabsorber kein Wasser absorbiert hat, und einen ausgefüllten Zustand hat, in dem der Superabsorber das Wasser absorbiert hat und in der die Hülle ein größeres Volumen als in dem Lagerungszustand umschließt. Durch das Vorsehen der Hülle ist es beispielsweise möglich, dass der Superabsorber als ein Granulat vorliegt. Es ist besonders bevorzugt, dass die Hülle eine Mehrzahl an Durchgangslöchern aufweist. Das Wasser kann durch die Durchgangslöcher in das Innere der Hülle gelangen und somit kann der Superabsorber das Wasser absorbieren. Der Superabsorber kann durch die Durchgangslöcher aber nicht nach außen gelangen. Alternativ oder zusätzlich ist bevorzugt, dass die Hülle einen Werkstoff aufweist oder aus dem Werkstoff besteht, der durchlässig für das Wasser ist. Durch ein Einstellen der Wasserdurchlässigkeit der Hülle, beispielsweise über die Anzahl und Größe der Durchgangslöcher, lässt sich zudem die Absorptions- und damit Ausdehnungsgeschwindigkeit des Superabsorbers kontrollieren.

Der Superabsorber liegt bevorzugt als ein Granulat vor.

Es ist bevorzugt, dass der Optikkörper eine Optikkörperaussparung aufweist, die an einem in einer Radialrichtung bezüglich der optischen Achse außen liegenden Rand des Optikkörpers eingebracht ist und eingerichtet ist, einen Teil des Superabsorbers aufzunehmen, wenn sich das Volumen des Superabsorbers vergrößert. Dadurch kann vermieden werden, dass die Vergrößerung des Volumens des Superabsorbers eine Verformung des Optikkörpers bewirkt, die zu einem Abbildungsfehler führen kann.

Die Intraokularlinse weist bevorzugt eine zweite Haptik auf, die eine dritte Teilhaptik und eine vierte Teilhaptik aufweist, die an dem Optikkörper befestigt sind und in der Axialrichtung nebeneinander angeordnet sind sowie einen Annäherungszustand und einen Beabstandungszustand haben, in dem die dritte Teilhaptik und die vierte Teilhaptik weiter voneinander entfernt als in dem Annäherungszustand sind, wobei der Superabsorber in der Axialrichtung zwischen der dritten Teilhaptik und der vierten Teilhaptik angeordnet ist und eingerichtet ist, sein Volumen durch eine Absorption von Wasser zu vergrößern und somit die dritte Haptik und die vierte Haptik von dem Annäherungszustand in den Beabstandungszustand zu bringen. Besonders bevorzugt ist die zweite Haptik symmetrisch bezüglich der optischen Achse zu der ersten Haptik ausgebildet. In dem Fall, dass die Intraokularlinse den Ring aufweist, ist es bevorzugt, dass der Ring sowohl den zwischen der ersten Teilhaptik und der zweiten Teilhaptik angeordneten Superabsorber als auch den zwischen der dritten Teilhaptik und der vierten Teilhaptik angeordneten Superabsorber aufweist. In einem anderen Fall kann die Intraokularlinse ein erstes Kissen, das an der ersten Haptik angeordnet ist, und ein zweites Kissen aufweisen, das an der zweiten Haptik angeordnet ist und im Abstand von dem ersten Kissen angeordnet ist.

Es ist bevorzugt, dass der Superabsorber eingerichtet ist, sein Volumen durch die Absorption von Wasser um mindestens das 50fache, insbesondere das 70fache oder das 90fache, zu vergrößern.

Superabsorber sind kommerziell erhältlich und werden beispielsweise in Windeln eingesetzt. Beispielsweise kann der Superabsorber ein Acrylsäurepolymer aufweisen. Das Acrylsäurepolymer kann mittels eines Quervernetzers quervernetzt sein. Der Quervernetzer kann beispielsweise ein Diacrylatester, ein Allylmethacrylat, ein Triallylamin und/oder ein Tetraallyloxyethan aufweisen. Als Initiatoren können beispielsweise Redox-Systeme wie K₂S₂O₈/Na₂S₂O₅ oder Wasserstoffperoxid/Ascorbat eingesetzt werden. Bei dem Acrylsäurepolymer kann es sich auch um ein Copolymer aufweisend Acrylsäure und/oder Natriumacrylat und Acrylamid handeln. In anderen Beispielen kann der Superabsorber ein Ethylen-Maleinsäureanhydrid-Copolymer, eine vernetzte Carboxymethylcellulose, Polyvinylalkohol-Copolymere und/oder ein vernetztes Polyethylenoxid aufweisen.

Die Intraokularlinse ist bevorzugt eine hydrophobe Intraokularlinse. Die hydrophobe Intraokularlinse wird herkömmlich an der Luft gelagert, so dass die Erfindung besonders relevant für die hydrophobe Intraokularlinse ist. Alternativ ist denkbar, dass die Intraokularlinse eine hydrophile Intraokularlinse ist. In dem Fall, dass die hydrophile Intraokularlinse in einer Flüssigkeit gelagert wird, die Wasser aufweist, sind Maßnahmen zu ergreifen, um die Absorption des Wassers durch den Superabsorber während der Lagerung zu unterbinden. Beispielsweise kann die Hülle wasserundurchlässig ausgebildet sein und nach dem Einsetzen der Intraokularlinse in den Kapselsack eines Auges kann die Hülle aufgeschnitten oder anderweitig eröffnet werden, wie beispielsweise durch eine Punktion und/oder eine Beaufschlagung mit Laserstrahlung.

Es ist bevorzugt, dass die erste Haptik und/oder die zweite Haptik in der Draufsicht plattenförmig oder C-förmig ausgebildet sind.

Im Folgenden wird anhand der beigefügten schematischen Zeichnungen die Erfindung näher erläutert. Es zeigen
Figur 1 eine perspektivische Ansicht einer ersten Ausführungsform einer Intraokularlinse,
Figur 2 eine perspektivische Ansicht einer zweiten Ausführungsform der Intraokularlinse,
Figur 3 einen Querschnitt durch eine dritte Ausführungsform der Intraokularlinse in einem Annäherungszustand von Teilhaptiken der Intraokularlinse und
Figur 4 den Querschnitt aus Figur 3 in einem Beabstandungszustand der Teilhaptiken.

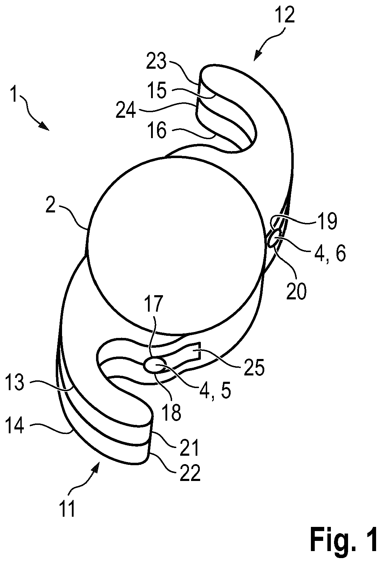

Wie es aus Figuren 1 bis 4 ersichtlich ist, weist eine Intraokularlinse 1 einen Optikkörper 2, der eine optische Achse 3 aufweist, eine erste Haptik 11 und einen Superabsorber 4 auf. Die erste Haptik 11 weist eine erste Teilhaptik 13 und eine zweite Teilhaptik 14 auf, die an dem Optikkörper 2 befestigt sind, in einer Axialrichtung 9 bezüglich der optischen Achse 3 nebeneinander angeordnet sind sowie einen Annäherungszustand und einen Beabstandungszustand haben, in dem die erste Teilhaptik 13 und die zweite Teilhaptik 14 weiter voneinander entfernt als in dem Annäherungszustand sind. Der Superabsorber 4 ist in der Axialrichtung 9 zwischen der ersten Teilhaptik 13 und der zweiten Teilhaptik 14 angeordnet und eingerichtet, sein Volumen durch eine Absorption von Wasser zu vergrößern und somit die erste Teilhaptik 13 und die zweite Teilhaptik 14 von dem Annäherungszustand in den Beabstandungszustand zu bringen. Der Superabsorber 4 kann dazu in einer Umfangsrichtung 8 bezüglich der optischen Achse 3 an der gleichen Stelle wie die erste Teilhaptik 13 und die zweite Teilhaptik 14 angeordnet sein. Bei dem Wasser kann es sich beispielsweise um bereits in dem Auge befindliches Kammerwasser handeln. Alternativ oder zusätzlich ist denkbar, dass das Wasser mittels einer Irrigation während einer Kataraktbehandlung in das Auge eingebracht wird.

Die Intraokularlinse 1 kann eine zweite Haptik 12 aufweisen, die eine dritte Teilhaptik 15 und eine vierte Teilhaptik 16 aufweist, die an dem Optikkörper 2 befestigt sind und in der Axialrichtung 9 nebeneinander angeordnet sind sowie einen Annäherungszustand und einen Beabstandungszustand haben, in dem die dritte Teilhaptik 15 und die vierte Teilhaptik 16 weiter voneinander entfernt als in dem Annäherungszustand sind, wobei der Superabsorber 4 in der Axialrichtung 9 zwischen der dritten Teilhaptik 15 und der vierten Teilhaptik 16 angeordnet ist und eingerichtet ist, sein Volumen durch eine Absorption von Wasser zu vergrößern und somit die dritte Teilhaptik 15 und die vierte Teilhaptik 16 von dem Annäherungszustand in den Beabstandungszustand zu bringen. Der Superabsorber 4 kann dazu in der Umfangsrichtung 8 an der gleichen Stelle wie die dritte Teilhaptik 15 und die vierte Teilhaptik 16 angeordnet sein.

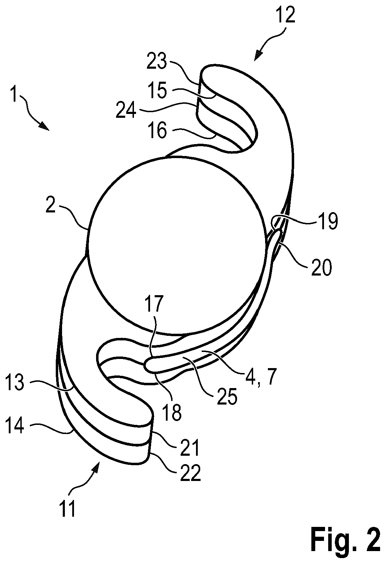

Die erste Haptik 11 und die zweite Haptik 12 können an voneinander abgewandten Seiten des Optikkörpers 2 angebracht sein, siehe Figuren 1 und 2. Zudem ist denkbar, dass die zweite Haptik 12 symmetrisch bezüglich der optischen Achse 3 zu der ersten Haptik 11 ausgebildet ist.

Ferner ist es denkbar, dass der Optikkörper eine Optikkörperaussparung 25 aufweist, die an einem in einer Radialrichtung 10 bezüglich der optischen Achse 3 außen liegenden Rand des Optikkörpers 2 eingebracht ist. Die Optikkörperaussparung 25 ist eingerichtet, einen Teil des Superabsorbers 4 aufzunehmen, wenn sich das Volumen des Superabsorbers 4 vergrößert.

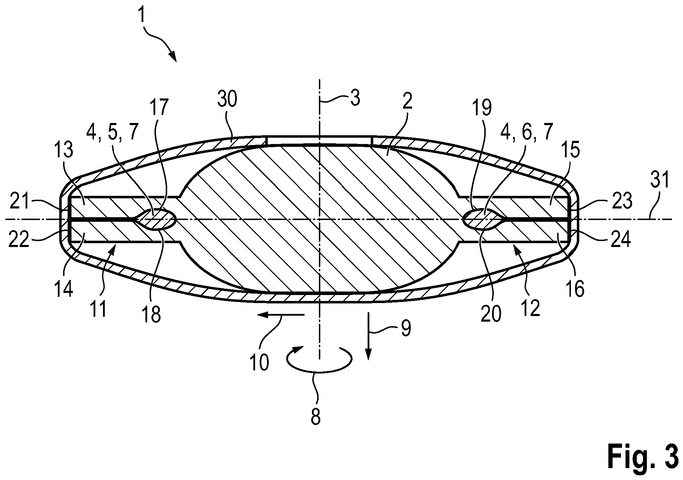

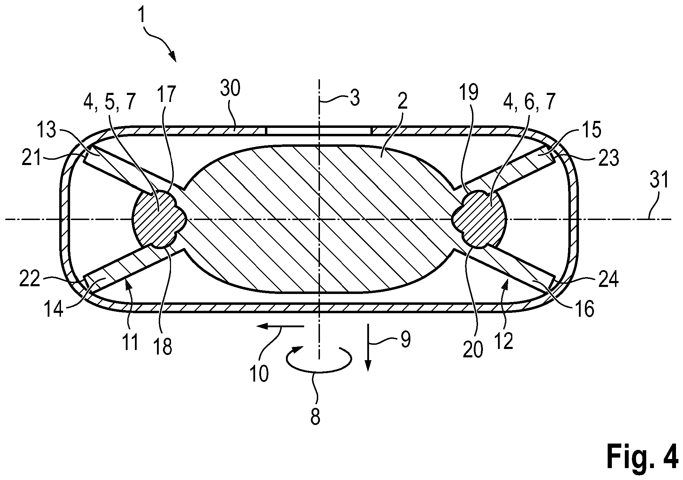

Figuren 3 und 4 zeigen, dass die Intraokularlinse 1 eine gedachte Trennebene 31 aufweisen kann, deren Normale parallel zu der Axialrichtung 9 ist, wobei die erste Teilhaptik 13 im Wesentlichen vollständig auf einer Seite der Trennebene 31 angeordnet ist und die zweite Teilhaptik 14 im Wesentlichen vollständig auf einer anderen Seite der Trennebene 31 angeordnet ist. Zudem kann die dritte Teilhaptik 15 im Wesentlichen vollständig auf der einen Seite der Trennebene 31 angeordnet sein und die vierte Teilhaptik 16 kann im Wesentlichen vollständig auf der anderen Seite der Trennebene 31 angeordnet sein. Beispielsweise sind die erste Teilhaptik 13 und die dritte Teilhaptik 15 im Wesentlichen vollständig auf der einen Seite der Trennebene 31 angeordnet und die zweite Teilhaptik 14 und die vierte Teilhaptik 16 im Wesentlichen vollständig auf der einen Seite der Trennebene 31 angeordnet, wenn die Intraokularlinse 1 sich in einem Entspannungszustand befindet, in dem die Intraokularlinse 1 frei von einer mechanischen Spannung ist. Zudem können die erste Teilhaptik 13 und die dritte Teilhaptik 15 im Wesentlichen vollständig auf der einen Seite der Trennebene 31 angeordnet sein und die zweite Teilhaptik 14 und die vierte Teilhaptik 16 können im Wesentlichen vollständig auf der anderen Seite der Trennebene 31 angeordnet, wenn die erste Teilhaptik 13, die zweite Teilhaptik 14, die dritte Teilhaptik 15 und die vierte Teilhaptik 16 sich in dem Beabstandungszustand befinden, vergleiche Figur 4.

Der Superabsorber 4 kann beispielsweise eingerichtet sein, sein Volumen durch die Absorption von Wasser um mindestens das 50fache, insbesondere das 70fache oder das 90fache, zu vergrößern.

In Figuren 3 und 4 ist die Intraokularlinse 1 in einem Kapselsack 30 eines Auges angeordnet dargestellt. In Figur 3 befinden sich die erste Teilhaptik 13 und die zweite Teilhaptik 14 in dem Annäherungszustand und die dritte Teilhaptik 15 und die vierte Teilhaptik 16 befinden sich in dem Annäherungszustand, wohingegen in Figur 4 sich die erste Teilhaptik 13 und die zweite Teilhaptik 14 in dem Beabstandungszustand befinden und die dritte Teilhaptik 15 und die vierte Teilhaptik 16 sich in dem Beabstandungszustand befinden. Es ist denkbar, dass in dem Annäherungszustand der Kapselsack 30 den Optikkörper 2 berührt, wie es in Figur 3 dargestellt ist. In dem Beabstandungszustand wird der Kapselsack 30 von der ersten Haptik 11 und der zweiten Haptik 12 aufgespannt, so dass eine in dem Kapselsack 30 angeordnete Flüssigkeit besser zirkulieren kann, wodurch sich die Wahrscheinlichkeit einer Bildung eines Nachstars verringert. Zudem zeigt Figur 4, dass in dem Beabstandungszustand die erste Teilhaptik 13, die zweite Teilhaptik 14, die dritte Teilhaptik 15 und die vierte Teilhaptik 15 zumindest teilweise in der Axialrichtung 9 über den Optikkörper 2 hinaus ragen können. Insbesondere können die erste Teilhaptik 13 und die dritte Teilhaptik 15 auf einer Seite des Optikkörpers 2 entgegen der Axialrichtung 9 über die maximale axiale Erstreckung des Optikkörpers 2 hinaus ragen und die zweite Teilhaptik 14 und die vierte Teilhaptik 16 auf einer anderen Seite des Optikkörpers 2 in der Axialrichtung 9 über die maximale axiale Erstreckung des Optikkörpers 2 hinaus ragen. Dadurch kann erreicht werden, dass der Kapselsack 30 den Optikkörper 2 nicht mehr berührt, wodurch sich die Wahrscheinlichkeit der Bildung des Nachstars besonders stark verringern lässt.

Figuren 3 und 4 zeigen, dass die erste Teilhaptik 13 ein erstes Längsende 21 aufweisen kann, das dem Optikkörper 2 abgewandt angeordnet ist, und die zweite Teilhaptik 14 ein zweites Längsende 22 aufweisen kann, das dem Optikkörper 2 abgewandt angeordnet ist, wobei in dem Annäherungszustand die erste Teilhaptik 13 in einem Bereich des ersten Längsendes 21 die zweite Teilhaptik 14 in einem Bereich des zweiten Längsendes 22 kontaktieren kann, wobei in dem Beabstandungszustand die erste Teilhaptik 13 in dem Bereich des ersten Längsendes 21 beabstandet von der zweiten Teilhaptik 14 in dem Bereich des zweiten Längsendes 22 angeordnet sein kann. In analoger Weise kann die dritte Teilhaptik 15 ein drittes Längsende 23 aufweisen, das dem Optikkörper 2 abgewandt angeordnet ist, und die vierte Teilhaptik 16 kann ein viertes Längsende 24 aufweisen, das dem Optikkörper 2 abgewandt angeordnet ist, wobei in dem Annäherungszustand die dritte Teilhaptik 15 in einem Bereich des dritten Längsendes 23 die vierte Teilhaptik 16 in einem Bereich des vierten Längsendes 24 kontaktieren kann, wobei in dem Beabstandungszustand die dritte Teilhaptik 15 in dem Bereich des dritten Längsendes 23 beabstandet von der vierten Teilhaptik 16 in dem Bereich des vierten Längsendes 24 angeordnet sein kann.

Wie es aus Figuren 1 bis 4 ersichtlich ist, kann die erste Teilhaptik 13 an ihrer der zweiten Teilhaptik 14 zugewandten Seite eine erste Aussparung 17 aufweisen, in der der Superabsorber 4 angeordnet ist. Zudem kann die zweite Teilhaptik 14 an ihrer der ersten Teilhaptik 13 zugewandten Seite eine zweite Aussparung 18 aufweisen, in der der Superabsorber 4 angeordnet ist. Die erste Aussparung 17 und die zweite Aussparung 18 können jeweils einen halbkreisförmigen Querschnitt haben, die in dem Annäherungszustand der ersten Teilhaptik 13 und der zweiten Teilhaptik 14 zusammen einen kreisförmigen Querschnitt bilden. In analoger Weise kann die dritte Teilhaptik 15 an ihrer der vierten Teilhaptik 16 zugewandten Seite eine dritte Aussparung 19 aufweisen, in der der Superabsorber 4 angeordnet ist. Zudem kann die vierte Teilhaptik 16 an ihrer der dritten Teilhaptik 15 zugewandten Seite eine vierte Aussparung 20 aufweisen, in der der Superabsorber 4 angeordnet ist. Die dritte Aussparung 19 und die vierte Aussparung 20 können jeweils einen halbkreisförmigen Querschnitt haben, die in dem Annäherungszustand der dritten Teilhaptik 15 und der vierten Teilhaptik 16 zusammen einen kreisförmigen Querschnitt bilden.

Figur 1 zeigt, dass die Intraokularlinse 1 ein erstes Kissen 5, das den Superabsorber 4 in dem Bereich der ersten Haptik 11 aufweist, und ein zweites Kissen 6 aufweisen kann, das in einem Abstand von dem ersten Kissen 5 angeordnet ist und den Superabsorber 4 in dem Bereich der zweiten Haptik 12 aufweist. In dem Annäherungszustand kann das erste Kissen 5 vollständig in der ersten Aussparung 17 und der zweiten Aussparung 18 angeordnet sein und das zweite Kissen 6 kann vollständig in der dritten Aussparung 19 und der vierten Aussparung 20 angeordnet sein.

Figur 2 zeigt, dass die Intraokularlinse 1 einen Ring 7 aufweisen kann, der sich in einer Umfangsrichtung 8 bezüglich der optischen Achse 3 vollumfänglich um den Optikkörper 2 erstreckt und der den Superabsorber 4, insbesondere den gesamten Superabsorber 4, aufweist.

Es ist denkbar, dass die Intraokularlinse 1 eine Hülle aufweist, die für das Wasser durchlässig ist und in der der Superabsorber 4, insbesondere als ein Granulat, angeordnet ist, wobei die Hülle einen Lagerungszustand, in dem der Superabsorber 4 kein Wasser absorbiert hat, und einen ausgefüllten Zustand hat, in dem der Superabsorber 4 das Wasser absorbiert hat und in der die Hülle ein größeres Volumen als in dem Lagerungszustand umschließt. Damit die Hülle für das Wasser durchlässig ist, kann die Hülle beispielsweise eine Mehrzahl an Durchgangslöchern aufweisen und/oder einen Werkstoff aufweisen oder aus dem Werkstoff bestehen, der durchlässig für das Wasser ist.

### Bezugszeichenliste

1 Intraokularlinse
2 Optikkörper
3 optische Achse
4 Superabsorber
5 erstes Kissen
6 zweites Kissen
7 Ring
8 Umfangsrichtung
9 Axialrichtung
10 Radialrichtung
11 erste Haptik
12 zweite Haptik
13 erste Teilhaptik
14 zweite Teilhaptik
15 dritte Teilhaptik
16 vierte Teilhaptik
17 erste Aussparung
18 zweite Aussparung
19 dritte Aussparung
20 vierte Aussparung
21 erstes Längsende
22 zweites Längsende
23 drittes Längsende
24 viertes Längsende
25 Optikkörperaussparung
30 Kapselsack
31 Trennebene

## Patentansprüche

1. Intraokularlinse mit einem Optikkörper (2), der eine optische Achse (3) aufweist, einer ersten Haptik (11), die eine erste Teilhaptik (13) und eine zweite Teilhaptik (14) aufweist, die an dem Optikkörper (2) befestigt sind, **dadurch gekennzeichnet, dass** die erste Teilhaptik (13) und die zweite Teilhaptik (14) in einer Axialrichtung (9) bezüglich der optischen Achse (3) nebeneinander angeordnet sind sowie einen Annäherungszustand und einen Beabstandungszustand haben, in dem die erste Teilhaptik (13) und die zweite Teilhaptik (14) weiter voneinander entfernt als in dem Annäherungszustand sind, und einem Superabsorber (4), der in der Axialrichtung (9) zwischen der ersten Teilhaptik (13) und der zweiten Teilhaptik (14) angeordnet ist und der eingerichtet ist, sein Volumen durch eine Absorption von Wasser zu vergrößern und somit die erste Teilhaptik (13) und die zweite Teilhaptik (14) von dem Annäherungszustand in den Beabstandungszustand zu bringen.

2. Intraokularlinse gemäß Anspruch 1, wobei in dem Beabstandungszustand die erste Teilhaptik (13) und die zweite Teilhaptik (14) zumindest teilweise in der Axialrichtung (9) über den Optikkörper (2) hinaus ragen.

3. Intraokularlinse gemäß Anspruch 1 oder 2, wobei die Intraokularlinse (1) eine gedachte Trennebene (31) aufweist, deren Normale parallel zu der Axialrichtung (9) ist, wobei die erste Teilhaptik (13) im Wesentlichen vollständig auf einer Seite der Trennebene (31) angeordnet ist und die zweite Teilhaptik (14) im Wesentlichen vollständig auf einer anderen Seite der Trennebene (31) angeordnet ist.

4. Intraokularlinse gemäß einem der Ansprüche 1 bis 3, wobei die erste Teilhaptik (13) an seiner der zweiten Teilhaptik (14) zugewandten Seite eine erste Aussparung (17) aufweist, in der der Superabsorber (4) angeordnet ist.

5. Intraokularlinse gemäß einem der Ansprüche 1 bis 4, wobei die Intraokularlinse (1) einen Ring (7) aufweist, der sich in einer Umfangsrichtung (8) bezüglich der optischen Achse (3) vollumfänglich um den Optikkörper (2) erstreckt und der den Superabsorber (4) aufweist.

6. Intraokularlinse gemäß einem der Ansprüche 1 bis 5, wobei die Intraokularlinse (1) eine Hülle aufweist, die für das Wasser durchlässig ist und in der der Superabsorber (4) angeordnet ist, wobei die Hülle einen Lagerungszustand, in dem der Superabsorber (4) kein Wasser absorbiert hat, und einen ausgefüllten Zustand hat, in dem der Superabsorber (4) das Wasser absorbiert hat und in der die Hülle ein größeres Volumen als in dem Lagerungszustand umschließt.

7. Intraokularlinse gemäß einem der Ansprüche 1 bis 6, wobei der Optikkörper (2) eine Optikkörperaussparung aufweist, die an einem in einer Radialrichtung (10) bezüglich der optischen Achse (3) außen liegenden Rand des Optikkörpers (2) eingebracht ist und eingerichtet ist, einen Teil des Superabsorbers (4) aufzunehmen, wenn sich das Volumen des Superabsorbers (4) vergrößert.

8. Intraokularlinse gemäß einem der Ansprüche 1 bis 7, wobei die Intraokularlinse (1) eine zweite Haptik (12) aufweist, die eine dritte Teilhaptik (15) und eine vierte Teilhaptik (16) aufweist, die an dem Optikkörper (2) befestigt sind und in der Axialrichtung (9) nebeneinander angeordnet sind sowie einen Annäherungszustand und einen Beabstandungszustand haben, in dem die dritte Teilhaptik (15) und die vierte Teilhaptik (16) weiter voneinander entfernt als in dem Annäherungszustand sind, wobei der Superabsorber (4) in der Axialrichtung (9) zwischen der dritten Teilhaptik (15) und der vierten Teilhaptik (16) angeordnet ist und eingerichtet ist, sein Volumen durch eine Absorption von Wasser zu vergrößern und somit die dritte Teilhaptik (15) und die vierte Teilhaptik (16) von dem Annäherungszustand in den Beabstandungszustand zu bringen.

9. Intraokularlinse gemäß einem der Ansprüche 1 bis 8, wobei der Superabsorber (4) als ein Granulat vorliegt.

10. Intraokularlinse gemäß einem der Ansprüche 1 bis 9, wobei der Superabsorber (4) eingerichtet ist, sein Volumen durch die Absorption von Wasser um mindestens das 50fache zu vergrößern.

## Claims

1. Intraocular lens having an optics body (2) which comprises an optical axis (3), a first haptic (11) comprising a first partial haptic (13) and a second partial haptic (14) both attached to the optics body (2), **characterized in that** the first partial haptic (13) and the second partial haptic (14) are arranged next to one another in an axial direction (9) with respect to the optical axis (3) and have a convergence state and a divergence state in which the first partial haptic (13) and the second partial haptic (14) are further apart from one another than in the convergence state, and a superabsorber (4) arranged between the first partial haptic (13) and the second partial haptic (14) in the axial direction (9) and configured to increase its volume by absorbing water and thus transfer the first partial haptic (13) and the second partial haptic (14) from the convergence state to the divergence state.

2. Intraocular lens according to Claim 1, wherein, in the divergence state, the first partial haptic (13) and the second partial haptic (14) protrude at least partially beyond the optics body (2) in the axial direction (9).

3. Intraocular lens according to Claim 1 or 2, wherein the intraocular lens (1) has an imaginary separation plane (31), the normal of which is parallel to the axial direction (9), with substantially the entirety of the first partial haptic (13) being arranged on one side of the separation plane (31) and substantially the entirety of the second partial haptic (14) being arranged on another side of the separation plane (31).

4. Intraocular lens according to any of Claims 1 to 3, wherein the first partial haptic (13) has a first recess (17), in which the superabsorber (4) is arranged, on its side facing the second partial haptic (14).

5. Intraocular lens according to any of Claims 1 to 4, wherein the intraocular lens (1) has a ring (7) which extends fully around the optics body (2) in a circumferential direction (8) with respect to the optical axis (3) and which comprises the superabsorber (4).

6. Intraocular lens according to any of Claims 1 to 5, wherein the intraocular lens (1) comprises a cover which is permeable to water and in which the superabsorber (4) is arranged, with the cover having a storage state, in which the superabsorber (4) has absorbed no water, and a filled state, in which the superabsorber (4) has absorbed the water and in which the cover encloses a greater volume than in the storage state.

7. Intraocular lens according to any of Claims 1 to 6, wherein the optics body (2) has an optics body recess which is introduced in an outer edge of the optics body (2) in a radial direction (10) with respect to the optical axis (3) and which is configured to accommodate some of the superabsorber (4) once the volume of the superabsorber (4) has increased.

8. Intraocular lens according to any of Claims 1 to 7, wherein the intraocular lens (1) comprises a second haptic (12) comprising a third partial haptic (15) and the fourth partial haptic (16) both attached to the optics body (2) and arranged next to one another in the axial direction (9) and also having a convergence state and a divergence state in which the third partial haptic (15) and the fourth partial haptic (16) are further apart from one another than in the convergence state, with the superabsorber (4) being arranged between the third partial haptic (15) and the fourth partial haptic (16) in the axial direction (9) and being configured to increase its volume by absorbing water and hence transfer the third partial haptic (15) and the fourth partial haptic (16) from the convergence state to the divergence state.

9. Intraocular lens according to any of Claims 1 to 8, wherein the superabsorber (4) is present as a granulate material.

10. Intraocular lens according to any of Claims 1 to 9, wherein the superabsorber (4) is configured to increase its volume at least 50-fold by absorbing water.

## Revendications

1. Lentille intraoculaire, comprenant un corps optique (2) qui présente un axe optique (3), une première haptique (11) qui présente une première haptique partielle (13) et une deuxième haptique partielle (14) qui sont fixées au corps optique (2),
**caractérisée en ce que** la première haptique partielle (13) et la deuxième haptique partielle (14) sont disposées l'une à côté de l'autre dans une direction axiale (9) par rapport à l'axe optique (3) et présentent un état de rapprochement et un état d'éloignement dans lequel la première haptique partielle (13) et la deuxième haptique partielle (14) sont plus éloignées l'une de l'autre qu'à l'état de rapprochement, et par un superabsorbant (4) qui est disposé dans la direction axiale (9) entre la première haptique partielle (13) et la deuxième haptique partielle (14), et qui est conçu pour augmenter son volume par une absorption d'eau et pour faire passer ainsi la première haptique partielle (13) et la deuxième haptique partielle (14) de l'état de rapprochement à l'état d'éloignement.

2. Lentille intraoculaire selon la revendication 1, dans laquelle à l'état d'éloignement, la première haptique partielle (13) et la deuxième haptique partielle (14) dépassent au moins partiellement du corps optique (2) dans la direction axiale (9).

3. Lentille intraoculaire selon la revendication 1 ou 2, dans laquelle la lentille intraoculaire (1) présente un plan de séparation imaginaire (31) dont la normale est parallèle à la direction axiale (9), dans laquelle la première haptique partielle (13) est disposée sensiblement entièrement sur un côté du plan de séparation (31) et la deuxième haptique partielle (14) est disposée substantiellement entièrement sur un autre côté du plan de séparation (31).

4. Lentille intraoculaire selon l'une quelconque des revendications 1 à 3, dans laquelle la première haptique partielle (13) présente sur son côté tourné vers la deuxième haptique partielle (14) un premier évidement (17) dans lequel est disposé le superabsorbant (4).

5. Lentille intraoculaire selon l'une quelconque des revendications 1 à 4, dans laquelle la lentille intraoculaire (1) présente un anneau (7) qui s'étend par rapport à l'axe optique (3) dans une direction circonférentielle (8) par rapport à l'axe optique (3) intégralement autour du corps optique (2) et qui présente le superabsorbant (4).

6. Lentille intraoculaire selon l'une quelconque des revendications 1 à 5, dans laquelle la lentille intraoculaire (1) présente une enveloppe qui est perméable à l'eau et dans laquelle est disposé le superabsorbant (4), dans laquelle l'enveloppe présente un état de conservation dans lequel le superabsorbant (4) n'a pas absorbé de l'eau et un état plein dans lequel le superabsorbant (4) a absorbé l'eau et dans lequel l'enveloppe comprend un volume plus grand qu'à l'état de conservation.

7. Lentille intraoculaire selon l'une quelconque des revendications 1 à 6, dans laquelle le corps optique (2) présente un évidement de corps optique qui est pratiqué dans un bord du corps optique (2), situé à l'extérieur dans une direction radiale par rapport à l'axe optique (3), et qui est conçu pour recevoir une partie du superabsorbant (4) lorsque le volume du superabsorbant (4) augmente.

8. Lentille intraoculaire selon l'une quelconque des revendications 1 à 7, dans laquelle la lentille intraoculaire (1) présente une deuxième haptique (12) qui présente une troisième haptique partielle (15) et une quatrième haptique partielle (16) qui sont fixées au corps optique (2) et sont disposées l'une à côté de l'autre dans la direction axiale (9) et présentent un état de rapprochement et un état d'éloignement dans lequel la troisième haptique partielle (15) et la quatrième haptique partielle (16) sont plus éloignées l'une de l'autre qu'à l'état de rapprochement, dans laquelle le superabsorbant (4) est disposé dans la direction axiale (9) entre la troisième haptique partielle (15) et la quatrième haptique partielle (16) et est conçu pour augmenter son volume par une absorption d'eau et pour faire passer ainsi la troisième haptique partielle (15) et la quatrième haptique partielle (16) de l'état de rapprochement à l'état d'éloignement.

9. Lentille intraoculaire selon l'une quelconque des revendications 1 à 8, dans laquelle le superabsorbant (4) est présent sous forme de granulés.

10. Lentille intraoculaire selon l'une quelconque des revendications 1 à 9, dans laquelle le superabsorbant (4) est conçu pour augmenter au moins 50 fois de volume du fait de l'absorption d'eau.
